(19) Europäisches Patentamt
European Patent Office
Office européen des brevets

(11) Numéro de publication: **0 216 646**
**B1**

(12) **FASCICULE DE BREVET EUROPEEN**

(45) Date de publication du fascicule du brevet: **10.10.90**

(51) Int. Cl.$^5$: **C 07 C 291/04,** A 61 K 31/13

(21) Numéro de dépôt: **86401630.8**

(22) Date de dépôt: **22.07.86**

(54) **Nouveau dérivé oxyde de la NN-diméthyl éthylamine, son procédé de préparation et les compositions pharmaceutiques en renfermant.**

(30) Priorité: **22.07.85 FR 8511152**
**16.10.85 FR 8515448**

(43) Date de publication de la demande:
**01.04.87 Bulletin 87/14**

(45) Mention de la délivrance du brevet:
**10.10.90 Bulletin 90/41**

(84) Etats contractants désignés:
**AT BE CH DE FR GB IT LI LU NL SE**

(56) Documents cités:
**FR-A-2 384 495**
**FR-M- 5 498**

(73) Titulaire: **Albert ROLLAND S.A. Société dite**
**49, Rue St-André-des-Arts**
**F-75006 Paris (FR)**

(72) Inventeur: **Labaune, Jean-Pierre**
**10 rue des Grands Cèdres**
**F-77310 Ponthierry-Moulignon (FR)**

(74) Mandataire: **Burtin, Jean-François**
**Cabinet GEFIB 55 Rue Boissonade**
**F-75014 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 216 646 B1

**Description**

La présente invention concerne un nouveau dérivé de la NN-di-méthyl éthylamine et en particulier N-oxydé.

Elle concerne tout particulièrement le N-oxyde de 2,2-bis phénoxy NN-diméthyl éthylamine de formule

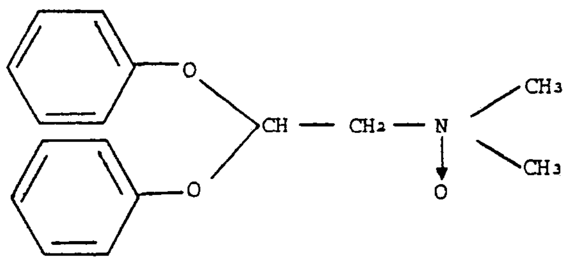

Elle concerne également les sels du N-oxyde avec un acide minéral ou organique et en particulier avec un acide fort comme un chlorhydrate, un bromhydrate ou un sulfate.

Le N-oxyde de 2,2-bis phénoxy NN-diméthyl éthylamine est une base faible et donne des sels essentiellement avec des acides forts non réducteurs.

Ces composés selon l'invention sont préparés selon un procédé qui consiste à soumettre la 2,2-bis phénoxy NN-diméthyl éthylamine ou un de ses sels à l'action d'une péroxyde minéral ou organique dans un solvant inerte. De préférence, le péroxyde sera le perhydrol. Il pourra être également un hydropéroxyde d'alcanol comme le péroxyde de terbutyle; un peroxyde de cétone comme le peroxyde d'hexafluoro acétone; un péracide comme l'acide p. nitroperbenzoïque ou l'acide N-chloroperbensoïque.

Le solvant du milieu réactionnel est un solvant inerte comme le méthanol, l'éthanol, un hydrocarbure aromatique comme le toluène ou le xylène, ou bien encore un amide NH-disubstitué comme le diméthyl formamide ou le diméthyl acétamide.

Le N-oxyde de 2,2-bis phénoxy NN-diméthyl éthylamine et ses sels possèdent les propriétés caractéristiques des autres anti-dépresseurs, sur les tests pharmacologiques classiques. Ils inhibent l'effet hypothermisant de la réserpine, et de l'apomorphine, ils augmentent la toxicité de groupe induite par injection de yohimbine et ils antagonisent le test du désespoir chez la souris.

La 2,2-bis phénoxy NN-diméthyl éthylamine est déjà décrite dans la littérature ainsi que ses sels (brevet spécial de Médicament 5498M). Les sels de 2,2-bis phénoxy NN-diméthyl éthylamine sont décrits comme possédant des propriétés anti-dépressives. Les essais menés par la société demanderesse ont mis en évidence le fait que ces produits manifestaient une toxicité aiguë appréciable chez la souris qui permet de fixer une dose léthale moyenne vers 800 mg/kg.

Au contraire, le N-oxyde objet de la présente demande possède une toxicité très faible tout en gardant une activité comparable et il n'a pas été possible de déterminer à des doses acceptables pour les animaux, une dose léthale moyenne.

En outre, le N-oxyde manifeste une action antagoniste sur le test à l'apomorphine et une action positive vis-à-vis de la toxicité de groupe à la yohimbine nettement plus marquées que celles manifestées par la 2,2-bis phénoxy NN-diméthyl éthylamine.

En d'autres termes, le N-oxyde est plus actif que le composé de l'art antérieur dont il dérive, tout en étant nettement moins toxique.

Le brevet français 2.384.495 a été cité pour montrer l'équivalence qui peut exister en une pentane diamine N-acylée et son N-oxyde. On peut simplement constater que dans une famille chimique tout à fait différente, les deux types de composés se comportant un pharmacologie d'une manière similaire. Au contraire, dans la série des bis phénoxy éthylamine, la 2,2-bis phénoxy NN-diméthyl éthylamine et son N-oxyde ont été des propriétés pharmacologiques bien différentes. Il est fréquent dans la littérature de noter qu'un N-oxyde est moins toxique ou à peu près du même niveau de toxicité quel'amine tertiaire dont il dérive mais cette diminution de la toxicité est aussi le résultat d'une baisse de l'activité pharmacologique chez l'animal. Dans le cas de la bis phénoxy NN-diméthyl éthylamine, non seulement le N-oxyde objet de l'invention est beaucoup moins toxique, mais en plus, il est sensiblement plus actic que l'amine tertiaire de départ.

En raison de leurs propriétés anti-dépressives, ils trouvent un emploi en médecine humaine ou vétérinaire dans le traitement des états dépressifs, des psychoses d'involution, des états maniaco-dépressifs, des dépressions réactionnelles ou comportementales. Ces molécules étant très peu toxiques, leur marge thérapeutique est très important et ils peuvent être administrés sans crainte des effets secondaires (sècheresse de la bouche, troubles extrapyramidaux) qui peuvent survenir avec les antidépresseurs du type tricyclique.

La posologie en N-oxyde de 2,2-bis phénoxy NN-diméthyl éthylamine pourra varier largement en fonction du poids et de l'âge du sujet, de l'indication thérapeutique, de l'ancienneté de la maladie et de la voie d'administration.

Elle s'échelonne de 0.05 g à 0.500 g par prise unitaire et de 0.100 g à 0.500 g par jour chez l'adulte.

En vue de l'administration thérapeutique, le N-oxyde de 2,2-bis phénoxy NN-diméthyl amine ou ses sels d'addition seront présentés sous forme de compositions pharmaceutiques. Celles-ci renferment ledit N-oxyde ou un de ses sels en association ou en mélange avec un excipient ou un véhicule inerte non-toxique pharmaceutiquement-acceptable.

L'excipient ou le véhicule inerte est un de ceux qui conviennent pour l'administration par voie parentérale, buccale, rectale ou percutanée. On pourra citer à cet égard les amidons traités ou non, la cellulose, les celluloses o-alcoylées, le carbonate de calcium, le stéarate de magnésium ou le talc; l'eau ou les solutés physioligiques; le beurre de cacao ou les stéarates de polyéthylène glycols; les solvants polaires comme l'alcool benzylique ou le diméthylsulfoxyde.

La réalisation des compositions pharmaceutiques s'effectue selon les méthodes habituelles de la pharmacotechnie, notamment en vue de la réalisation des comprimés nus ou enrobés, des dragées, des gélules, des capsules, des gouttes, des solutés injectables ou buvables, des suppositoires et des solutions pour l'application percutanée.

Les exemples suivants illustrent l'invention. Ils ne la limitent en aucune facon.

Exemple I

Préparation du N-oxyde de 2,2-bis phénoxy NN-dimethyléthylamine

Dans un tricol de 100 ml muni d'une ampoule à brome, d'un thermomètre, d'un refrigérant, et contenant 5,15 g (0,02M) de N,-N-diméthyl-2,2-diphénoxy éthanamine dans 50 ml de méthanol, ou ajoute goutte à goutte 6 ml d'eau oxygénée à 30%.

Après 1/2 heure d'agitation à température ambiante, on chauffe à 35°C durant 12 heures.

La méthanol est évaporé, l'eau est éliminée par azéotropie à l'éthanol. L'huile obtenue est reprise à l'éther, un solide blanc se forme. Après filtration et sèchage, on recueille 3,08 g de N-oxyde de NN-diméthyl-2,2-diphénoxy éthanamine qui fond à 104°C.

Rendement = 56,5%.

$R_f$ = 0,14 $CHCL_3/CH_3OH$ 9/1

R.M.N.: ($CDCl_3$) 10H (m; 6,8—7,3); 1H (t; 6,7); 2H (d; 3,7); 6H (s; 3,2)

R.M.N.: ($CDCl_3$) 10H (m; 6,8—7,3); 1H (t; 5,8);
(Médifoxamine) 2H (d; 2,8); 6H (s; 2,32).

Exemple II
Etude pharmacologique du N-oxyde de 2,2-bis phénoxy NN-diméthyl éthylamine

| PRODUIT | STRUCTURE CHIMIQUE | TOXICITÉ AIGUË SOURIS P.O. | HYPOTHERMIE RÉSERPINE 100 P.O. | HYPOTHERMIE APOMORPHINE 50 P.O. | | TOXICITÉ YOHIMBINE 100 P.O. | TEST DU DÉSESPOIR SOURIS 100 P.O. |
|---|---|---|---|---|---|---|---|
| | | | | 50 P.O. | 100 P.O. | | |
| MEDIFOXAMINE | 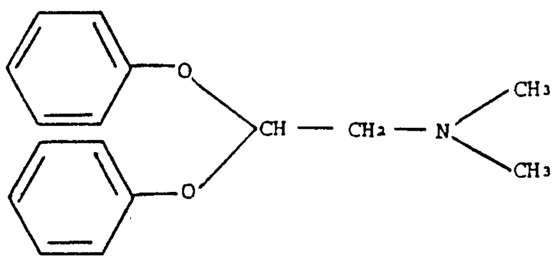 | 600 0/10<br>700 3/10<br>800 5/10 | + 2°5 * | + 1°1 * | + 0°9 * | 5/10 | 60% * |
| N-oxyèe | 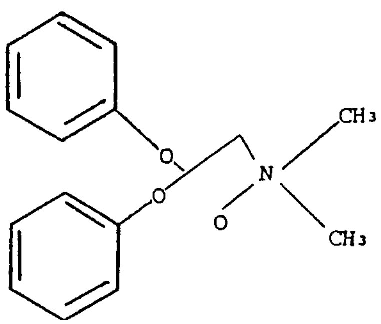 | 500 0/10<br>1000 1/10 | + 2° * | + 0°7 | + 1°6 * | 7/10 | 51% * |

**Revendications**

1. Lie N-oxyde de la 2,2-bis phénoxy NN-diméthyléthylamine de fomule

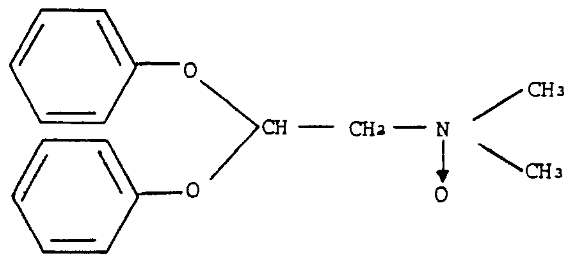

2. Les sels du N-oxyde selon la revendication 1 avec un acide minéral ou organique.

3. Un procédé d'obtention du composé selon la revendication 1 dans lequel on soumet la 2,2-bis phénoxy NN-diméthyl éthyl amine ou un de ses sels à l'action d'un péroxyde minéral ou organique dans un solvant inerte.

4. Les compositions pharmaceutiques renfermant à titre de principe actif un composé selon l'une des revendications 1 ou 2 en association ou en mélange avec un excipient ou un véhicule inerte non-toxique pharmaceutiquement-acceptable.

5. Les compositions pharmaceutiques selon la revendication 4 dans lesquelles l'excipient ou le véhicule sont un de ceux qui conviennent pour l'administration parentérale, buccale, rectale ou percutanée.

6. Les compositons pharmaceutiques selon la revendication 4 dans lesquelles la quantité de principe actif varie de 0,05 à 0,500 g par prise unitaire.

**Patentansprüche**

1. N-oxyd des 2,2-bis phenoxy NN-dimethylethylamins der Formula

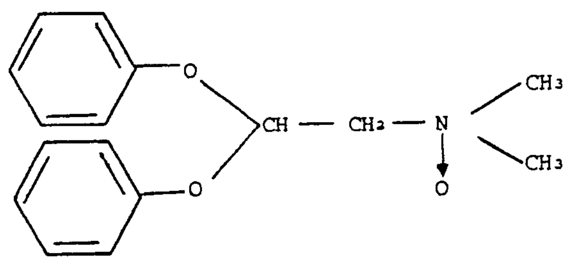

2. Die Salz mit einer anorganischen oder organischen Säure des N-oxyds nach Anspruch 1.

3. Verfahren zur Herstellung der Verbindung nach Anspruch 1 worin man zu die Einwirkung eines anorganischen oder organischen Peroxyd in einen inerten Lösungsmittel 2,2-bis phenoxy NN-dimethyl ethylamin oder eine Salz davon, unterwirft.

4. Die pharmazeutische Zubereitungen die als Wirkstoff eine Verbindung nach einer der Ansprüche 1 oder 2 enthält, in Verbindung oder Vermischung mit einem inerten, nicht-toxischen pharmazeutischverträglichen Träger oder Vehikel.

5. Die pharmazeutische Zubereitungen nach Anspruch 4 worin der Träger oder das Vehikel, eine der für parenterale, orale, rektale oder perkutane Verabreichung, eignet sind.

6. Die pharmazeutische Zubereitungen nach Anspruch 4, worin der Anteil an Wirkstoff von 0.05 bis 0.500 g per einzeln Dosis beträgt.

**Claims**

1. 2,2-bis phenoxy NN-dimethylethylamine N-oxyde having the formula

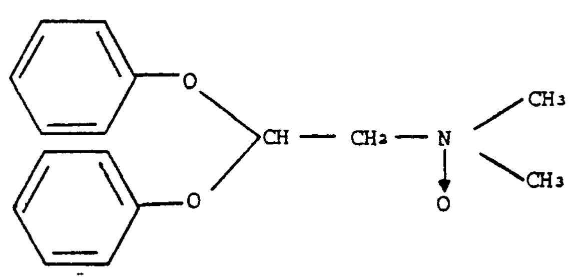

2. The acid addition salts with a mineral or organic acid of the N-oxyde according to claim 1.

3. A process for producing the compound according to claim 1 wherein the 2,2-bis phenoxy NN-dimethylethylamine or a salt thereof is reacted with a mineral or organic peroxyde in an inert solvent.

4. The pharmaceutical compositions which contain as active ingredient a compound according to any of claims 1 and 2 in admixture or conjunction with an inert non toxic pharmaceutically-acceptable vehicle or carrier.

5. The pharmaceutical compositions according to claim 4 wherein the carrier or the vehicle is one of those suitable for parenteral, oral, rectal or percutaneous ways of administration.

6. The pharmaceutical compositions according to claim 4 wherein the amount of active ingredient ranges from 0.05 to 0.500 g per unit dosage.